Europäisches Patentamt

European Patent Office

Office européen des brevets

(11) Veröffentlichungsnummer: **0 330 902**

**A2**

(12) **EUROPÄISCHE PATENTANMELDUNG**

(21) Anmeldenummer: **89102504.1**

(22) Anmeldetag: **14.02.89**

(51) Int. Cl.⁴: **G01N 33/577** , //**C12P21/00, C12N5/00,G01N33/569**

Patentanspruch für folgenden Vertragsstaat: ES

(30) Priorität: **24.02.88 DE 3805716**

(43) Veröffentlichungstag der Anmeldung:
**06.09.89 Patentblatt 89/36**

(84) Benannte Vertragsstaaten:
**AT BE CH DE ES FR GB IT LI LU NL SE**

(71) Anmelder: **BEHRINGWERKE**
**Aktiengesellschaft**
**Postfach 1140**
**D-3550 Marburg 1(DE)**

(72) Erfinder: **Dopatka, Hans-Detlef, Dr.**
**Heinrich-Heine-Strasse 9**
**D-3550 Marburg(DE)**

(74) Vertreter: **Meyer-Dulheuer, Karl-Hermann, Dr.**
**et al**
**HOECHST Aktiengesellschaft Postfach 3540**
**D-6121 Wiesbaden(DE)**

(54) **Verwendung monoklonaler Antikörper (mAK) als Durchführungskontrolle für immunometrische Teste.**

㊄ Es wird gezeigt, daß die Verwendung homologer mAK als Kontrolle in immunometrischen Testen zum Nachweis spezifischer Antikörper vorteilhaft ist

EP 0 330 902 A2

## Verwendung monoklonaler Antikörper (mAK) als Durchführungskontrolle für immunometrischen Teste

Die Erfindung betrifft die Verwendung von monoklonalen Antikörpern (mAK) geeigneter Spezifität als Durchführungskontrolle in immunometrischen Testen, die den Nachweis von spezifischen Antikörpern zum Ziel haben, z.B. in Enzyme-linked-Immunosorbent Assays (ELISA's), Radioimmunoassays (RIA's), Fluoreszenz-Immunoassays (FIA's), Lumineszenz-Immunoassays (LIA's) oder änlichen Testverfahren. Im folgenden wird die Erfindung an ELISA's beschrieben, weil ELISA's derzeit den größten Anwendungsbereich besitzen, ohne daß dadurch eine Beschränkung auf dieses Testverfahren zu verstehen ist.

Die Anforderungen von staatlichen Zulassungsbehörden oder vorgeschalteten, meinungsbildenden Gremien an eine "ideale" Testkontrolle in ELISA's, für die Bundesrepublik Deutschland zuletzt in "Richtlinien für die Durchführung von Enzym-Immuno-Assays bzw. Enzym-Liganden-Assays", Lab. med. 10, 391-392 (1986), zusammengefaßt, werden ständig größer. Folgende Richtlinien sind von Diagnostica-Herstellern mit den z.Zt. üblichen Testkontrollen auf Basis polyklonaler Antikörper nur schwer zu erfüllen:

- Angabe von Kriterien zur Überprüfung der Verwendbarkeit der Reagenzien durch den Anwender mittels Angabe der maximal zulässigen Meßergebnisabweichungen (Variationsbreite), bezogen auf die unterschiedlichen Abschnitte der Standardkurve.
- Bei Tests mit Ja/Nein-Aussage: Angabe des Meßbereichs (Variationsbreite), in dem die eingesetzten Kontrollen liegen müssen, wenn die Reagenzien ordnungsgemäß reagieren.
- Angabe der maximal zulässigen Standardabweichungen bzw. relativen Standardabweichungen (Variationskoeffizienten) der verschiedenen Standardkurvenbereiche.
- Bei Tests mit Ja/Nein-Aussage: Angaben über die Ergebnisse von Studien zur analytischen Empfindlichkeit und diagnostischen Sensitivität des Tests. Ferner Angaben, wann das Ergebnis als "sicher positiv", als "sicher negativ" oder als "zweifelhaft" anzusehen ist.

Nun besteht der Wunsch seitens der Anwender, auch bei ELISA's für den Nachweis spezifischer Antikörper quantitative Aussagen aus einer einzelnen Patientenprobe zu machen, wodurch innerhalb eines insgesamt verbesserten Testsystems der "Kontrollanteil" ebenfalls verbessert werden muß.

Die Fachwelt war bisher der Meinung, daß die in o.g. Testen notwendige Kontrolle am besten durch "Kontrollsubstanzen" erreicht werden kann, die den zu testenden Körperflüssigkeiten - in der Regel Seren -möglichst ähnlich sind. Diese "Kontrollsubstanzen" werden aus hochtitrigen Antiseren von humanen Spendern bzw. Spendertieren hergestellt, enthalten ein Gemisch polyklonaler Antikörper und sind so den zu testenden Proben äquivalent. Zusätzlich wurde es als Vorteil angesehen, damit zugleich eine natürliche "Matrix" als Teil dieser Kontrolle einzusetzen, um so - allerdings schwankende und schlecht reproduzierbare - Matrixeffekte in den Proben durch die Kontrolle mitzuerfassen.

Die bisher vorhandenen mAK, überwiegend murinen Ursprungs, werden diagnostisch entweder zu Antigen-Nachweisen oder zur Fixierung von Antigen-Reagenz an die feste Phase eingesetzt. Wir haben gefunden, daß mAK humanen, porcinen, bovinen u. a. Ursprungs mit geeigneter Spezifität als Kontrolle in den eingangs genannten Testen verwendbar sind, da damit die Kontrolle bei Ein-Punkt-Messungen verbessert wird und insbesondere folgende Vorteile verbunden sind.

1. Bisher werden geeignete Testkontrollen aus vorgetesteten und ausgesuchten individuellen Einzelblutspenden (Plasmaspenden) hergestellt. Daraus ergibt sich eine nach oben begrenzte Chargengröße mit von Charge zu Charge unterschiedlichen Affinitäten, die jeweils als Summe der verschiedenen Einzelaffinitäten des in dieser Spende vorhanden "Cocktails" von polyklonalen Antikörpern resultieren. Diese unterschiedlichen Affinitäten der polyklonalen Kontrollen führen zu unterschiedlichen Testwerten, (Lehtonen, O.-P. und Eerola, E. (1982), J. Immunol. Meth. 54, 233-240), was die Kontrollfunktion beeinträchtigen kann. Verwendung geeigneter mAK erlaubt die Herstellung von Kontrollen theoretisch unbegrenzter Chargengröße mit von Charge zu Charge gleicher Affinität, was zusätzlich den Prüfaufwand des Herstellers drastisch reduziert.

2. In der Regel ist bei Antikörper-nachweisenden ELISA's die Festphase mit einem von Charge zu Charge wechselnden Gemisch verschiedener Antigene, beispielsweise eines Virus, beschichtet. Kontrollen auf Basis polyklonaler Antikörper können dann sehr unterschiedlich auf eine wechselnde Zusammensetzung des Antigenmix an der Festphase reagieren. Kontrollen auf Basis von mAK zeigen diese Schwankungen nicht, sofern die zur Test-Durchführung erforderlichen Antigene ausreichend vorhanden sind und weisen darüberhinaus deren Fehlen in der Festphase zuverlässig nach.

3. Auch bei ELISA's mit gentechnologisch hergestellten, definierten Beschichtungsantigenen kann die Verwendung von Kontrollen auf Basis polyklonaler Antikörper Probleme verursachen. So ist beispielsweise beim Nachweis von Antikörpern gegen das Humane Cytomegalovirus (HCMV) bekannt, daß die neutralisierenden Antikörper einer Untersuchungsprobe gegen den sogenannten gp 58-Antigenanteil gerichtet sind. Sämtliche z.Z. bei HCMV-Antikörpernachweisen bekannten Testkontrollen auf Basis polyklonaler Antikörper enthalten aber Antikörper, von denen nur ein von Charge zu Charge wechselnder Anteil mit dem gp 58 reagiert mit entsprechender Beeinträchtigung der Kontrollfunktion.

4. Es ist von Vorteil, wenn die immunologisch reaktive Komponente der Kontrolle nicht durch die von Untersuchungsprobe zu Untersuchungsprobe möglichen, unterschiedlichen Störsubstanzen (Matrices) belastet ist, denn nur dies macht die zu kontrollierende Antigen-Antikörper-Reaktion repräsentativ. Hierbei handelt es sich um ein ernstes Problem, dem z.B. die WHO mit der Schaffung eines kaum realisierbaren "Matrix-Standards" auf andere Weise zu begegnen sucht (WHO/BS/85.1462, Geneva, 12.-18. November 1985).
Bei den gängigen, kommerziellen ELISA's kann obiger Forderung nur teilweise durch ein besonders hohes Vorverdünnen mühsam aufgefundener, hochtitriger Einzelspenden entsprochen werden. Kontrollen auf Basis von mAK können mit definierter Matrix hergestellt werden.

5. Bei biologischen Präparaten sollte jegliches Infektionsrisiko z.B. durch HIV- oder Hepatitis-Viren ausgeschlossen werden. Dies ist bei den üblicherweise zur Herstellung der Testkontrollen verwendeten Blutspenden trotz vorhandener Prüftests nicht in jedem Fall gewährleistet. Gerade in der frühen HIV-Infektionsphase kann es mit diesen Tests falsch negative Befunde geben, besonders bei isolierten HIV-2-Infektionen oder wenn bestimmte Rheumafaktoren in der Untersuchungsprobe enthalten sind.

Die Summe der aufgelisteten Forderungen erfüllen nur mAK, weil sie mit spezifischen Eigenschaften in definierter Weise herstellbar sind. Diese mAK können auch sehr gut als Substandard von einem gegebenenfalls vorhandenen Nationalen oder Internationalen Antikörper-Standard etabliert werden.

## Beispiele

Humane monoklonale Antikörper (hmAK) unterschiedlicher Virusspezifität werden im folgenden in den verschiedenen antikörpernachweisenden Enzygnost®-Testen (Behringwerke AG) der Humandiagnostik zur Verwendung als Kontrollen untersucht. Bei veterinärdiagnostischen Testen z.B. Enzygnost Aujeszky, Enzygnost Rinderleukose sind entsprechende, geeignete porcine mAK bzw. bovine mAK verwendbar. Ferner ist durch die Auswahl der Immunglobulinklasse (z.B. IgG, IgM, IgA) bzw. der Subklasse (IgG$_1$ oder IgG$_2$) die Verwendung in den betreffenden Testsystemen möglich.

## 1. Isolierung geeigneter hmAK gegen Rubella-Virus

Blut eines seropositiven Spenders wird über einen Ficoll® Paque (Deutsche Pharmacia GmbH) Dichtegradienten zentrifugiert und die Blutlymphozyten isoliert. Die Zellen werden auf eine Zellkonz. von $2 \times 10^6$/ml mit RPMI 1640 + 10 % FCS (Firma Flow) verdünnt.

Mit Epstein Barr Virus (EBV)-haltigem Kulturüberstand werden die B-Lymphozyten immortalisiert. Durch Zugabe von 0,5 ug/ml Cyclosporin A hemmt man die T-Zellen.

Nach Auswachsen der transformierten Zellen wird der Kulturüberstand jedes Näpfchens im Enzygnost Rubella auf Anwesenheit spezifischer Antikörper untersucht. Bei positivem Befund werden die Zellen mit einer humanen Tumor-B-Zell-Linie (z.B. GM 1500 6TG OUA$^+$ beschrieben in JAMES, K., BELL, G.Th. J. Imm. Meth. 100 (1987), S. 40) oder einer Maus-Myelom-Zelle (z.B. SP2 beschrieben in PINTUS, C., RANSOM, J. H,. EVANS, C.H. J. Imm. Meth. 61 (1983), 195-200) fusioniert.

Die resultierenden Hybride werden wiederum mit einem ELISA auf Antikörperspezifität getestet.

Anschließend werdend die pos. Zellen kloniert (Grenzverdünnung 1 Zelle/Näpfchen). Nach mehreren Reklonierungen (mind. 2x) erhält man eine stabile human IgG produzierende Zelle, die als Quelle für die gewünschten humanen mAK (hmAK) dienen kann.

Die Bestimmung des Antigens, gegen das die Antikörper gerichtet sind, erfolgt durch "Westernblotting". Bei diesen Untersuchungen zeigte sich, daß die Antikörper des Hybridoms 7H4 gegen das große Hüll-Protein (E1) des Röteln-Virus gerichtet sind.

## 2. Isolierung geeigneter hmAK (IgG) gegen Varizella-Zoster Virus (VZV) und andere Antigene

Die Gewinnung hmAK gegen Varizella-Zoster-Virus erfolgt in identischer Weise wie in Beispiel 1, wobei für Screening und Austestung der Spezifität Enzygnost-Varizella/Zoster verwendet wird. In entsprechender Abwandlung können hmAK gegen andere gewünschte Antigene erhalten werden.

## 3. Isolierung von mAK anderer Spezies

a) Porcine mAK können wie von F. Buchegger et al. (1987) J. of the Nat. Canc. Inst. 79, 337-342 beschrieben, bei Einsatz der betreffenden Antigene, z.B. Aujeszky-Virus, hergestellt werden

b) Bovine mAK können unter entsprechender Modifikation wie unter (a) bzw. ANDERSON D.V: et al, Vet. Immunol. Immunopath 15 (1987), 223-237, sowie TUCKER, EM. et al., Anim. Genet. 18 (1987), 29-40 und RAYBOULD, T. J.G., et al., Am.J.Vet.Res. 46 (1985), 426-427 beschrieben hergestellt werden.

c) Murine mAK lassen sich wie von Köhler und Milstein (G. Köhler und C. Milstein (1975) Nature 256, 495) beschrieben erzeugen.

## 4. Verwendung des anti-Rubella hmAK 7H4 als pos. Kontrolle im Enzygnost Rubella

Enzygnost Rubella (Behringwerke AG) ist ein kommerziell erhältlicher Sandwich-ELISA zum Nachweis von Rubella-spezifischen Antikörpern in menschlichen Seren. Dabei sind an die Vertiefungen einer Mikrotitrationsplatte (MTP) gereinigte Rubellavirusantigene gebunden, wobei nach Inkubation gegebenenfalls gebundene spezifische Antikörper in der Serumprobe durch ein Anti-human IgG- bzw. IgM Enzymkonjugat (hier: Anti-human IgG-Konjugat) nachgewiesen werden. Die obengenannte hmAK-Kontrolle wird wie eine Patientenprobe eingesetzt und ergibt in seriellen Verdünnungen jeweils um den Faktor 4 einen Endtiter von 1:30720.

Die Ergebnisse sind in Tab. 1 zusammengefaßt, wobei die mittlere Kolonne jeweils die Differenz der Absorption Antigenkolonne (1, 3, 5 .....11) minus Absorption Kontrollantigenkolonne (2, 4, 6, ....12) ist.

Im Unterschied zu einer polyklonalen Kontrolle sind die Extinktionen auf Kontrollantigen (= Beschichtung in den geradzahligen Kolonnen 2, 4 ... 12, wobei das Kontrollantigen durch analoge Reinigung von nicht Rubella-Virus infiziertem Zellmaterial erhalten wird) sämtlich $\leq 0.013$ $OD_{405}$ und damit um den Faktor 5 bis 10 geringer. Bei Einsatz dieser hmAK-Kontrolle in anderen Enzygnost®-Testen wie Enzygnost®-Cytomegalie, -VZV, -Anti-HSV, -Masern,- Parotitis beträgt der Titer <1:40, d.h. die Kreuzreaktivität beträgt nahe Null. Polyklonale Kontrollen beherbergen in der Regel Antikörper gegen viele Pathogene, was ihre Verwendung einschränkt.

Tabelle 1

| Enzygnost-Cytomegalie | | | Enzygnost-Varizella/Zoster | | | Enzygnost-Rubella | | | Enzygnost-Anti-HSV | | | Enzygnost-Masern | | | Enzygnost-Parotitis | | |
|---|---|---|---|---|---|---|---|---|---|---|---|---|---|---|---|---|---|
| Reihe 1 | | Reihe 2 | Reihe 3 | | Reihe 4 | Reihe 5 | | Reihe 6 | Reihe 7 | | Reihe 8 | Reihe 9 | | Reihe 10 | Reihe 11 | | Reihe 12 |
| .009 | ..... | =0.0 | =0.0 | ..... | .001 | 1.619 | 1.606 | 0.13 | .021 | .005 | .016 | .008 | .002 | .006 | .002 | =0.0 | .004 |
| .009 | ..... | =0.0 | =0.0 | ..... | .001 | 1.543 | 1.535 | .008 | .013 | .007 | .006 | .009 | .006 | .003 | =0.0 | ..... | =0.0 |
| .004 | ..... | =0.0 | =0.0 | ..... | =0.0 | 1.366 | 1.362 | .004 | .012 | .009 | .003 | .004 | ..... | =0.0 | =0.0 | ..... | =0.0 |
| .007 | .004 | .003 | =0.0 | ..... | .004 | 1.043 | 1.034 | .009 | .009 | .005 | .004 | .005 | ..... | =0.0 | =0.0 | ..... | =0.0 |
| .005 | ..... | =0.0 | .001 | =0.0 | .002 | .435 | .428 | .007 | .006 | .002 | .004 | .007 | ..... | =0.0 | =0.0 | ..... | =0.0 |
| .003 | .001 | .002 | .005 | .001 | .004 | .152 | .144 | .008 | .008 | .001 | .007 | .008 | .001 | .007 | =0.0 | ..... | .005 |
| .001 | ..... | =0.0 | =0.0 | ..... | .001 | .045 | .039 | .006 | =0.0 | ..... | =0.0 | .002 | ..... | =0.0 | =0.0 | ..... | =0.0 |
| .003 | ..... | =0.0 | =0.0 | ..... | =0.0 | .016 | .009 | .009 | .002 | ..... | =0.0 | .003 | .002 | .001 | =0.0 | ..... | .005 |

Titer: ◄ 1:40    ◄ 1:40    1:30720    ◄ 1:40    ◄ 1:40    ◄ 1:40

5. Verwendung des Anti-VZV hmAK 6/870204 im Enzygnost Varicella/Zoster

Analog zu Beispiel 4 ist die Verwendung des oben genannten hmAK 6/870204 im Enzygnost Varicella/Zoster. Die Ergebnisse sind in Tab. 2 zusammengefaßt.

Das üblicherweise verwendete Anti-Human-IgG-Konjugat weist diese hmAK also zuverlässig nach. Weiterhin ist die Affinität der an die Festphase gebundenen hmAK ausreichend, um die mehrmaligen Waschschritte bei der Durchführung des ELISA zu überstehen.

Tabelle 2

| Enzygnost-Cytomegalie | | | Enzygnost-Varizella/Zoster | | | Enzygnost-Rubella | | | Enzygnost-Anti-HSV | | | Enzygnost-Masern | | | Enzygnost-Parotitis | | |
|---|---|---|---|---|---|---|---|---|---|---|---|---|---|---|---|---|---|
| Reihe 1 | Reihe 2 | | Reihe 3 | Reihe 4 | | Reihe 5 | Reihe 6 | | Reihe 7 | Reihe 8 | | Reihe 9 | Reihe 10 | | Reihe 11 | Reihe 12 | |
| .117 | .034 | .083 | 2.156 | 2.067 | .089 | .106 | .024 | .002 | .120 | .023 | .097 | .084 | =0.0 | .073 | .048 | .007 | .081 |
| .082 | .016 | .066 | 2.419 | 3.351 | .069 | .077 | .006 | .071 | .076 | =0.0 | .088 | .070 | =0.0 | .082 | .040 | =0.0 | .068 |
| .070 | .009 | .061 | 2.317 | 2.256 | .061 | .069 | .004 | .065 | .061 | =0.0 | .078 | .065 | =0.0 | .081 | .057 | .001 | .056 |
| .065 | .003 | .062 | 2.022 | 1.960 | .062 | .069 | .003 | .066 | .062 | =0.0 | .081 | .061 | =0.0 | .068 | .057 | =0.0 | .057 |
| .964 | .003 | .061 | 1.269 | 1.209 | .069 | .069 | .004 | .065 | .089 | =0.0 | .090 | .061 | =0.0 | .070 | .057 | =0.0 | .057 |
| .063 | .005 | .058 | .538 | .477 | .061 | .068 | .003 | .065 | .060 | =0.0 | .087 | .066 | =0.0 | .070 | .053 | .093 | .055 |
| .061 | .005 | .056 | .199 | .139 | .060 | .065 | .002 | .063 | .069 | =0.0 | .122 | .070 | .001 | .069 | .061 | =0.0 | .062 |
| .061 | =0.0 | .062 | .097 | .037 | .060 | .067 | .005 | .062 | .059 | =0.0 | .143 | .067 | =0.0 | .068 | .061 | =0.0 | .065 |

Titer < 1:40   1:122880   < 1:40   < 1:40   < 1:40   < 1:40

EP 0 330 902 A2

Ansprüche

1. Verfahren zur Durchführung immunometrischer Tests, die zum Nachweis von spezifischen Antikörpern dienen, dadurch gekennzeichnet, daß als Testkontrolle ein oder mehrere monoklonale Antikörper (mAK) verwendet werden.

2. Verfahren nach Anspruch 1, dadurch gekennzeichnet, daß der oder die verwendeten mAK dieselbe Spezifität wie die nachzuweisenden Antikörper besitzen.

3. Verfahren nach Anspruch 1, dadurch gekennzeichnet, daß der oder die verwendeten mAK humanen, porcinen oder bovinen Ursprungs sind.

4. Verfahren nach Anspruch 3, dadurch gekennzeichnet, daß der oder die verwendeten humanen, porcinen oder bovinen mAK wahlweise IgG, IgM oder IgA-Struktur besitzen.

5. Testkit, der neben mit Antigen beschichteten Trägern und markierten Antikörpern einen oder mehrere monoklonale Antikörper nach Anspruch 1 enthält.

6. Testkit nach Anspruch 5, dadurch gekennzeichnet, daß der oder die enthaltenen mAK humane, porcine oder bovine mAK sind.

7. Verwendung eines oder mehrerer mAK als positive Kontrolle in immunometrischen Tests, die zum Nachweis spezifischer Antikörper dienen.

8. Verwendung eines oder mehrerer humaner mAK in immunometrischen Tests, die zum Nachweis von spezifischen humanen Antikörpern dienen.

9. Verwendung eines oder mehrerer porciner mAK in immunometrischen Tests, die zum Nachweis von spezifischen Antikörpern des Schweines dienen.

10. Verwendung eines oder mehrerer boviner mAK in immunometrischen Tests, die zum Nachweis von spezifischen Antikörpern des Rindes dienen.

11. Verwendung eines oder mehrerer mAK nach Anspruch 1, dadurch gekennzeichnet, daß der oder die mAK als Substandard eines Nationalen oder Internationalen Antikörper-Standards kalibriert sind.

Patentansprüche für folgenden Vertragsstaat ES

1. Verfahren zur Durchführung immunometrischer Tests, die zum Nachweis von spezifischen Antikörpern dienen, dadurch gekennzeichnet, daß als Testkontrolle ein oder mehrere monoklonale Antikörper (mAK) verwendet werden.

2. Verfahren nach Anspruch 1, dadurch gekennzeichnet, daß der oder die verwendeten mAK dieselbe Spezifität wie die nachzuweisenden Antikörper besitzen.

3. Verfahren nach Anspruch 1, dadurch gekennzeichnet, daß der oder die verwendeten mAK humanen, porcinen oder bovinen Ursprungs sind.

4. Verfahren nach Anspruch 3, dadurch gekennzeichnet, daß der oder die verwendeten humanen, porcinen oder bovinen mAK wahlweise IgG, IgM oder IgA-Struktur besitzen.

5. Verfahren nach Anspruch 1, dadurch gekennzeichnet, daß der oder die mAK als Substandard eines Nationalen oder Internationalen Antikörper-Standards kalibriert sind.